# EUROPÄISCHE PATENTSCHRIFT

(11) **EP 1 180 062 B1**
(45) Veröffentlichungstag und Bekanntmachung des Hinweises auf die Patenterteilung: **10.03.2004**
(21) Anmeldenummer: 00943639.5
(22) Anmeldetag: 24.05.2000
(51) Int. Cl.: B01J 13/02, A61K 9/50, A61K 9/51, A61K 9/16, B01F 5/06

(54) **VERFAHREN ZUR HERSTELLUNG VON MORPHOLOGISCH EINHEITLICHEN MIKRO- UND NANOPARTIKELN MITTELS EINES MIKROMISCHERS**
METHOD FOR PRODUCING MORPHOLOGICALLY UNIFORM MICRO AND NANOPARTICLES USING MICROMIXERS
PROCEDE DE PRODUCTION DE MICROPARTICULES ET NANOPARTICULES MORPHOLOGIQUEMENT HOMOGENES A L'AIDE D'UN MICROMELANGEUR

(30) Priorität: 26.05.1999 DE 19925184
(43) Veröffentlichungstag der Anmeldung: 20.02.2002
(73) Patentinhaber: Schering Aktiengesellschaft, 13353 Berlin (DE)
(72) Erfinder: HILDEBRAND, Gesine, D-10555 Berlin (DE); TACK, Johannes, D-13595 Berlin (DE); HARNISCH, Stephan, D-10717 Berlin (DE)
(86) Internationale Anmeldenummer: PCT/DE2000/001677
(87) Internationale Veröffentlichungsnummer: WO 2000/072955

(56) Entgegenhaltungen:
- WO-A-97/30783
- DE-A- 19 545 257
- DE-A- 19 908 171
- DE-C- 19 540 292

## Beschreibung

Die Erfindung betrifft ein neues kontinuierliches Verfahren zur Herstellung von morphologisch einheitlichen Mikro- und Nanopartikeln mittels Mikromischer, die Verkapselung von Wirkstoffen mit diesem Verfahren, sowie nach diesem Verfahren hergestellte Partikel.

Der Anmeldung liegen die folgenden Begriffsbestimmungen zugrunde:
**Nanopartikel:** Partikel mit einer Teilchengröße von 1 nm -1000 nm
**Mikropartikel:** Partikel mit einer Teilchengröße von 1 µm -1000 µm
**Partikel:** Oberbegriff für Mikro- und Nanopartikel unabhängig von der Verteilung der partikelbildenden Substanz im Partikel
**Nanokapsel:** Nanopartikel mit Kem-Schale-Struktur, dadurch gekennzeichnet, daß die partikelbildende Substanz als hüllenbildende Kapselwand vorliegt.
**Mikrokapsel:** Mikropartikel mit Kern-Schale-Struktur, dadurch gekennzeichnet, daß die partikelbildende Substanz als hüllenbildende Kapselwand vorliegt.
**Kapseln:** Oberbegriff für Mikro- und Nanokapseln
**Partikelbildende Substanz:** Wandbildner bzw. Einbettungsmaterial von Partikeln oder reiner Wirkstoff
**Koazervation:** Überführung eines gelösten Polymers in eine polymerreiche, noch lösungsmittelhaltige Phase mittels Desolvatation. Die einfache Koazervation kann durch Aussalzen, Temperaturänderung, pH-Änderung oder Lösungsmittelzusatz bewirkt werden. Bei einer komplexen Koazervation wird die Desolvatation durch entgegengesetzt geladene Ionen oder Polymere ausgelöst. Das Koazervat als partikelbildende Substanz scheidet sich auf das zu verkapselnde Material ab
**Mikroverkapselung:** Verkapselung eines Wirkstoffes in ein Partikel.
**Lösungsmittelentzug:** Entfernung von organischen Lösungsmitteln durch Verdampfung und/oder Extraktion.
**Innere Phase:** I, disperse, dispergierte Phase.
**Äußere Phase:** A, Dispersionsmittel.
**Natürliche Substanzen:** Substanzen natürlichen Ursprungs, sowie naturidentische und naturanaloge Substanzen.
**Mikromischer:** Mischer mit einer Mischkammer mit ineinandergreifenden Mikrokanälen, in der mindestens zwei fluide Medien an mindestens einer Grenzfläche in Form von Flüssigkeitslamellen kleiner 1000 µm so in Kontakt gebracht werden, daß sich Flüssigkeitslamellen bilden, die alternierend aus den eingesetzten Medien bestehen und beim Aufsteigen im Auslaßkanal in eine kohärente und eine nicht kohärente Phase übergehen.
**Statischer Mischer:** kontinuierlich betreibbarer Mischer ohne bewegliche Einbauten.

Die bekannten Verfahren zur Herstellung von Mikro - oder Nanopartikeln können wie folgt eingeteilt werden:
1. Phasentrennverfahren - Koazervation
   - einfach
   - komplex
2. mechanisch-physikalische Verfahren
   - Sprühverfahren
   - Zentrifugalverfahren
   - hot-emulsion-Verfahren
3. Polymerisationsverfahren
   - Emulsionspolymerisation (Polymerisation innerhalb disperser Phase)
   - Grenzflächenpolymerisation (Polymerisation an Grenzfläche disperse Phase/Dispersionsmittel)
4. Verfahren über Polymerdispersionen
   - Hitzedenaturierung
   - Desolvatation
   - Lösungsmittelverdampfung (solvent evaporation)
   - Quervemetzung

Alle Verfahren werden diskontinuierlich betrieben und eignen sich zur Mikroverkapselung von Wirkstoffen in eine bioabbaubare synthetische Polymermatrix bzw. Copolymermatrix und/oder in natürliche Substanzen.

Aus der Literatur bekannte synthetische Polymere für diesen Zweck sind Polyamide, Polyanhydride, Polyester, Polyorthoester, Polyacetate, Polylactone, Polyorthocarbonate u. a.. Vor allem haben bisher Polylactid und Polylactid-coglycolid-Polymere Anwendung gefunden.
Als natürliche Substanzen zur Mikroverkapselung eignen sich zum Beispiel Fette und Proteine wie Gelatine und Albumin, sowie Polysaccharide und deren Derivate wie z.B. Stärke und Cellulose und deren Derivate, Alginate und Chitosan.

So sind z.B. aus US 4.675.189 (Syntex Inc.), US 4.835.139 (Debiopharm S.A.) und EP 302 582 B1 (Southem Research Inst.) pharmazeutische Zusammensetzungen wasserlöslicher Peptide und Proteine bekannt, die auf der Basis der Koazervation hergestellt wurden.

Die Nachteile dieses Verfahrens bestehen neben der Verwendung toxikologisch problematischer Mittel wie Dichlormethan, Heptan und Silikonöl auch darin, daß die verschiedenen durchzuführenden Verfahrensschritte nur einen diskontinuierlichen Betrieb zulassen.
Ein in EP-A 315875 (Hoechst AG) beschriebenes Verfahren zur Herstellung bioabbaubarer Mikropartikel von wasserlöslichen Peptiden und Proteinen basiert auf dem Sprühtrocknungsverfahren, bei dem eine wäßrige Peptid- oder Proteinlösung in einer organischen Polymerlösung emulgiert und diese Emulsion sprühgetrocknet wird. Auch dieses Verfahren ist nur diskontinuierlich durchführbar.

Nach dem "Solvent - Evaporation - Verfahren" hergestellte Mikropartikel sind in zwei kanadischen Patentanmeldungen CA 2.100.925 (Rhone-Merieux) und CA 2.099.941 (Tanabe Seiyaku Co.) beschrieben.
In DE 19545257 (Schering AG) wird ein diskontinuierliches Verfahren beschrieben, dass mittels einer "Induced Phase Transition" - Methode morphologisch einheitliche, nicht agglomerierende Mikrokapseln herstellt.

Üblicherweise werden bei den vorgenannten Methoden der Wirkstoff in einer organischen Polymerlösung gelöst, suspendiert oder direkt bzw. als wäßrige Lösung emulgiert. Nach Zugabe dieser Polymer/Wirkstoffdispersion zu einer zweiten wäßrigen Phase mit einer grenzflächenaktiven Substanz wird das Polymerlösungsmittel verdampft.

Diese Methode ist sehr variable und es werden normalerweise O/W, aber auch W/O oder komplexe W/O/W-Emulsionen hergestellt (Müller/Hildebrand: Pharmazeutische Technologie: Moderne Arzneiformen, Wiss. Verlagsgesellschaft, 2..Aufl., S. 243-258, 1998).

Essentieller Nachteil dieser Verfahren ist auch hier, daß es sich um diskontinuierliche Verfahren handelt. Im Labormaßstab erfolgt die Produktion z.B. im Becherglas, bei industrieller Herstellung bleibt die Produktionsweise erhalten, da lediglich das Ansatzgefäß vergrößert wird.

Herstellparameter wie z.B. die Dispergierzeit sind nicht direkt vom Labor- auf den Technikumsmaßstab übertragbar. Diese Unterschiede führen zu Schwierigkeiten beim Scaling-up.
Aufgrund eines großen Mischvolumens entstehen mit Großmischern während der Dispergierung im Medium stark unterschiedlich durchmischte Bereiche. Die Dispergierung ist inhomogen und es resultieren uneinheitliche Produkte.

In der EP 0 167 825 wird die Dispergierung z.B. mit hochtourigen Mischern wie Rotor-Stator-Systemen beschrieben. Nachteilig ist, daß uneinheitlich verteilte Leistungsdichten im Dispergiermedium auftreten und die Partikel dadurch uneinheitlich werden.

Die US-Patentschrift 5,188,837 beschreibt beispielsweise die Dispergierung durch Beschallung mittels Ultraschallstäben. Die Produkte sind aber oft mit Metall kontaminiert (z.B. mit Titan des Ultraschallstabes). Zusätzlich ist die Leistungsdichte sowohl um den Rührer als auch um den Kopf des Ultraschallstabes inhomogen, was zu Polydispersität der Partikel führt (Weyhers, H, Dissertationsschrift, Freie Universität Berlin, 1995).

Eine weitere Möglichkeit besteht in der Dispergierung mittels Hochdruckhomogenisation. Es können zwar in der Regel einheitliche Partikel hergestellt werden, doch die unter hohen Drücken auftretende Erosion am Kavitationsspalt kontaminiert das Produkt mit Metallionen. Zusätzlich können die bei Hochdruckhomogenisatoren auftretenden Temperaturspitzen empfindliche Wirkstoffe zersetzen. Die hohe Scherbelastung aufgrund des hohen Druckes (100 - 2000 bar) kann die Zersetzung von Makromolekülen (z.B. Albumin) zur Folge haben.

Eine Verbesserung der W/O/W-Technik wird in der Patentanmeldung WO 97/19676 beansprucht, indem beim Dispergierprozeß ein zusätzlicher Phaseninversionsprozeß induziert wird. Eine W/O Emulsion wird durch Rühren hergestellt, wobei die W-Phase den Wirkstoff enthält. Bei weiterem Zusatz wirkstofffreier Wasserphase erfolgt eine Phaseninversion zum W/O/W System, das unter Rühren konventionell weiterverarbeitet wird. Da immer noch zwei Dispergierprozesse involviert sind, bleiben die oben beschriebenen Probleme bestehen. Hinzu kommt jedoch, daß der Phaseninversionsprozeß zusätzlich noch kontrolliert werden muß.

Weiterhin werden Verfahren beschrieben, bei denen die Partikelmatrix in flüssiger Form in einer äußeren nicht mischbaren Phase direkt verteilt wird. Beispiele sind die Herstellung fester Lipidnanopartikel durch Rotor-Stator-Systeme, durch Ultrabeschallung oder durch Homogenisation (EP 0605497; Siekmann et al. Pharm. Pharmacol. Lett., 1 (1992) 123-126).

Weiterhin werden Arzneiformen beschrieben, in denen die partikelbildende Substanz reiner Wirkstoff ist. Die Herstellung von Partikeln aus reinen Wirkstoffen kann auch durch Hochdruckhomogenisation erfolgen (DE4440337) Generell gelten die oben beschriebenen Nachteile. Eine weitere Herstellmöglichkeit besteht in der Mahlung der Partikel (z.B. durch Perlmühlen wie bei NanoCrystals). Das Produkt ist aber durch den Abrieb der Mahlkugeln belastet (Buchmann et al., 42^{nd} Congress APV, 124, Mainz, Germany 1996).

Eine Herstellungsmethode für "Mikrokapseln" mit sehr schmaler Partikelgrößenverteilung mittels poröser Glasmembranen wird von Muramatsu et al. (J. Microencapsulation 1994, 11(2), pp 171-178) beschrieben. Bei diesem Verfahren wird die disperse Phase durch die Poren einer Glasmembran in ein zirkulierendes Dispersionsmittel gedrückt.
Dieses Verfahren zeichnet sich dadurch aus, daß nahezu monodisperse kolloidale Systeme erzeugt werden können, jedoch beträgt der Gehalt an disperser Phase in der Suspension max. 1% (m/m) und es ist bislang nur die Herstellung von Mikropartikeln beschrieben worden. Insbesondere eine kontinuierliche Herstellung von Mikrokapseln ist nicht möglich.

Ein Ansatz zur kontinuierlichen Herstellung von Mikropartikeln ist die Verwendung einer handelsüblichen Rotor-Stator-Durchflußzelle wie z.B. von Janke und Kunkel (Ika Labortechnik, Staufen, Deutschland) oder Silverson (Silverson Machined Limited, Chesham, United Kingdom ). Letztere wird z.B. in der PCT-Anmeldung WO 98/35654 beschrieben. Die oben diskutierten Rotor-Stator-Probleme bleiben erhalten. Speziell für die aseptische Produktion . ergeben sich zusätzlich Probleme bzgl. Keimfreiheit, da die bei diesen Rotoren erforderlichen Dichtungen anfällig für Keimbesatz sind.

Ein weiterer Ansatz zur kontinuierlichen Herstellung von "Mikrokugeln" ist in DE19908171 und EP 0 963 787 beschrieben, wobei eine disperse Phase durch "Mikrokanäle" nicht genannter Größe in eine kontinuierliche Phase gepreßt wird. Nachteilig bei diesem Verfahren ist das geringe Durchsatzvolumen, das, laut Kawakatsu et al. (J Chem Eng Jpn, 32(2), pp 241-44 (1999)), gewöhnlich bei 14 µl/min (!) disperser Phase liegt und somit für eine effiziente Anwendung in einem Produktionsprozeß nicht geeignet ist.

Die Schwierigkeit der Realisierung eines geeigneten großtechnischen Verfahrens zur Herstellung von Nanopartikeln wird auch dadurch belegt, daß bisher trotz mehr als 30 Jahren intensiver Forschung die Arzneiform Nanopartikel auf dem Markt nicht existiert. Neben technischen Problemen (Realisierung eines kontinuierlichen Prozesses, Kosten des Prozesses) kommt noch die oft fehlende Qualifizierungsfähigkeit der Anlagen dazu.

Zusammenfassend ergeben sich folgende Nachteile des Standes der Technik:
1. diskontinuierliche Produktionstechnik
2. Unterschiede in Produktionsbedingungen von Labor- und Technikumsmaßstab, dadurch Schwierigkeiten beim Scaling-up
3. inhomogene Mischbedingungen im Großansatz
4. uneinheitliche Produkte
5. Kontrollierbarkeit des Prozesses
6. Produktbelastung durch Produktionsprozeß (Kontamination)
7. Wirkstoffzersetzung
8. Kosten des Prozesses
9. Qualifizierungs- und Validierungsfähigkeit des Prozesses bzw. der Anlage

Aufgabe der Erfindung ist es, ein kontinuierliches Verfahren zur Herstellung morphologisch einheitlicher, nicht agglomerierender Mikro- und Nanopartikel ohne Verwendung von organischen Lösungsmitteln oder unter Verwendung von toxikologisch unbedenklichen Lösungsmitteln zu erhalten, das zur Mikroverkapselung von Wirkstoffen geeignet ist und das eine problemlose Überführung von einem Labor- auf einen Produktionsmaßstab ("Scaling-up") zuläßt. Weiterhin sollten die Herstellbedingungen keine Produktbelastung und Wirkstoffzersetzung zur Folge haben.

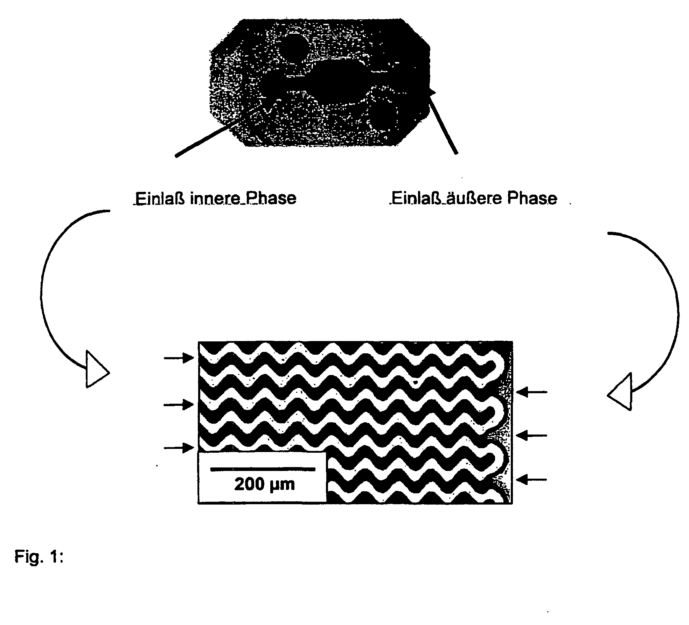

Die Aufgabe der Erfindung wird überraschend durch ein kontinuierliches Verfahren zur Herstellung von Mikro- oder Nanopartikeln aus bioabbaubaren synthetischen und/oder aus natürlichen Substanzen, dadurch gekennzeichnet, dass die Herstellung mittels eines Mikromischers erfolgt, der eine Mischkammer mit ineinandergreifenden Mikrokanälen enthält, in der Flüssigkeitslamellen der zu mischenden Flüssigkeiten periodisch zur Überlappung gebracht werden. Geeignete Mikromischer werden in Abb. 1 bis 4 dargestellt. Die erfindungsgemäß verwendeten Mikromischer basieren dabei auf einem einfachen, aber vielseitigen Multilamellar-Prinzip. Die Flüssigkeiten werden durch eine Lamellenschicht gepreßt, wobei sich die Schichtdicke der austretenden Flüssigkeit im Mikrometerbereich befindet.

Das zentrale Element eines solchen Mikromischers ist eine Mischkammer mit einer ineinandergreifenden Mikrokanalanordnung.

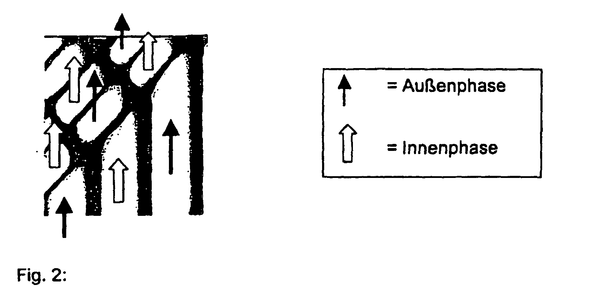

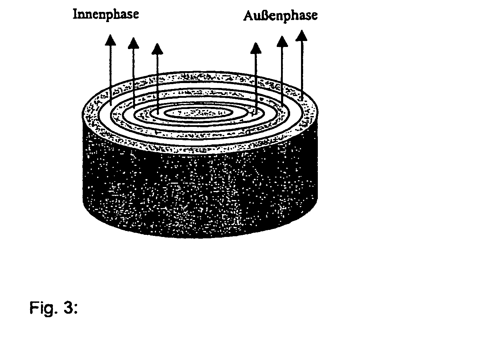

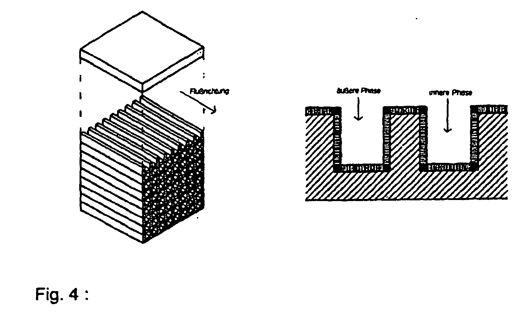

Beispiele für die erfindungsgemäß verwendeten Mikromischer sind:
- Mikromischer, Institut für Mikrotechnik Mainz GmbH, Mainz, Deutschland, (Fig.1: Mischerarray-Mischkammer (oben) mit Einlaß für die äußere und innere Phase, vergrößerter Ausschnitt aus dem Mischerarray (unten))
- Mikromischer, Siemens, Deutschland (Fig. 2)
- Mikrotube Mischer, Mesa Research Institute, Niederlande (Fig. 3: koaxiale Zuführung der Innen- und Außenphase)
- Mikromischer, Forschungszentrum Karlsruhe, Deutschland (Fig. 4)

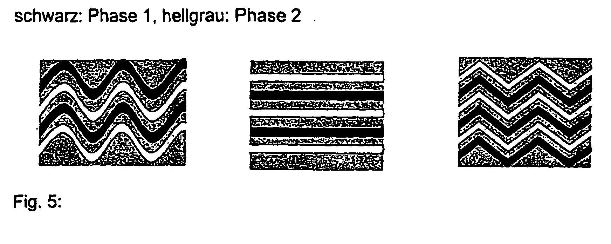

Die Wände der Mikrokanäle können unterschiedlich ausgeformt sein. Beispielsweise können die Wände gerade, zickzackförmig oder wellenförmig sein (Fig. 5: Beispiele für verschiedene Kanalformen in Mikromischem).

Die Mischkammer kann beispielsweise mittels einer Kombination aus Lithographie, Galvanoformung und Abformung (LIGA) hergestellt werden. Die Mikrokanalweite ist variabel und liegt zwischen 1-1000 µm, bevorzugt zwischen 2 und 500 µm und besonders bevorzugt zwischen 5 und 100 µm.

Die zu mischenden Flüssigkeiten werden drucklos oder bei Flüssigkeitsdrücken bis zu ca. 30 bar über Einlaßbohrungen dem Multikanalsystem zugeführt. Dies kann z. B. im Gegenfluß, Gleichfluß oder auch koaxial erfolgen. Dabei kommt es zur periodischen Überlappung der Flüssigkeitslamellen. Das Mischprodukt wird dabei nicht über die gesamte Oberfläche der Mischkammer abgezogen, sondern senkrecht oder parallel zur Flußrichtung über einen Auslaßkanal, der die völlige Durchdringung innerhalb einer definierten Kontaktzone erzwingt.

Mit diesem Mikromischem lassen sich überraschenderweise kontinuierlich Mikropartikel herstellen. Zusätzlich zeichnen sich die mit dem Verfahren gewonnenen Partikel überraschenderweise durch eine einheitliche Partikelgröße aus.

In der vorliegenden Erfindung wird durch den Einsatz von Mikromischem der Mischraum vom Maßstab von mehreren 100 Litern (z.B. Beco-Mix Mischer) auf ein Volumen von maximal wenigen cm³, in der Regel unter 1 cm³, mit einer Grundfläche von in der Regel weniger als 3 cm² reduziert. Es resultiert eine niedrige Leistungsdichte und die Produktbelastung wird minimiert.

Bei üblichen Flüssigkeitsdrücken von 1-2 bar können Durchflußvolumina von 1-1,5 L pro Stunde erreicht werden. Es können aber auch Drücke bis zu ca. 30 bar zum Pumpen der Flüssigkeiten durch die Mikrokänale des Mischers eingesetzt werden, bei geeigneter Konstruktion der Mischer auch höhere Drücke. Somit können die Durchflußvolumina entsprechend erhöht werden, daß heißt pro Stunde können mit einem Mischer weit mehr als 10 L Produkt erzeugt werden.

Durch Variation der Mikrokanalbreite und der Auslaßkanalbreite kann die Mischeinheit auf viele verschiedene Herstellparameter wie Flußrate, Druck und Flüssigkeitseigenschaften wie Viskosität angepaßt werden. Damit können Partikelgröße und -verteilung der hergestellten Mikropartikel in weiten Grenzen beeinflußt und kontrolliert werden.

Vorteil der Erfindung ist, daß ein Scaling-up ohne Veränderung der Herstellbedingungen erfolgen kann, das heißt die Produktqualität bleibt unverändert.
Durch einen parallelen Einsatz von Mikromischem läßt sich der Volumenstrom und damit der Durchsatz auf einfache Weise erhöhen. Alternativ können auch mehrere vorgefertigte Mischer-Arrays (bestehend aus z.B. 10 Einzelmischern) verwendet werden.
Ein Scaling-up vom Labor- auf einen Produktionsmaßstab läßt sich somit einfach durch die Erhöhung der Mikromischeranzahl erreichen (Numbering-up).

Die Zufuhr der Phasen erfolgt jeweils aus einem gemeinsamen Vorratsbehälter für sämtliche Mischer.
Die Durchmesser der Zufuhrleitungen sind so geregelt, daß bei jedem Mischer identische Druckverhältnisse und Strömungsgeschwindigkeiten herrschen. Selbst bei einem niedrigen Druck von 1-2 bar ergeben sich in einem Mischer-Array ca. 5 L Produkt pro Stunde, das heißt bereits bei Kombination von nur 10 Arrays einsteht ein Durchsatz von 50 L Partikeldispersion pro Stunde.

Die Erfindung eignet sich hervorragend zur aseptischen Produktion von Partikeln. Es können Mikromischer aus Metall eingesetzt werden (z.B. Stahl, Silber), die autoklaviert und sogar mit Hitze sterilisiert werden können. Die zugeführten Phasen können problemlos steril filtriert werden. Selbst bei geschmolzenen Lipiden ist Sterilfiltration unter Druck problemlos möglich. Bei der aseptischen Produktion werden die Förderpumpen vor die Sterilfilter geschaltet. Produktion im Mischer erfolgt dann unter aseptischen Bedingungen im Laminar-Air-Flow. Speziell der Einsatz von Silber-Mikromischern ist hier empfehlenswert, da zusätzlich noch der oligodynamische Effekt (List: Arzneiformenlehre, Wiss. Verlagsgesellschaft, 3.Aufl., S. 445, 1982) zum Tragen kommt.

Es sind die meisten zur Zeit noch diskontinuierlich betriebenen Verfahren auch mit einem Mikromischer auf den kontinuierlichen Betrieb umstellbar.

Die Mikropartikelbildung kann dabei-direkt in der Kontaktzone oder zeitlich verzögert auftreten. Die Mikro- bzw. Nanopartikelsuspension wird über den Auslaß abgezogen und kann gegebenenfalls weiter aufgearbeitet werden.

Die Verkapselung eines Wirkstoffes kann je nach dessen Löslichkeit in einfacher Weise erfolgen. Der Wirkstoff wird einfach, direkt oder gelöst in einem geeigneten Medium, in der partikelbildenden flüssigen Phase gelöst, suspendiert oder emulgiert.

Ist es erforderlich, den Wirkstoff oder eine Wirkstofflösung zu emulgieren, kann dies zweckmäßigerweise in einem vorgeschalteten Mischer, vorzugsweise Mikromischer oder durch eine zusätzliche Einlaßbohrung bewerkstelligt werden.

Ist die Herstellung einer organischen Lösung der partikelbildenden Substanz nicht vermeidbar, kann es erforderlich werden, die gewonnene Mikropartikeldispersion nachzuprozessieren.
Im einfachsten Fall läßt sich das Restlösungsmittel mittels einer cross-flow-Filtration oder eines Dünnschichtverdampfers (z.B. Sambay) im kontinuierlichen Betrieb entfernen.
Dünnschichtverdampfung und cross-flow-Filtration eignen sich zudem zur Entfernung von unverkapseltem Wirkstoff und/oder des/der Tenside.

Über die Einlaßbohrungen werden dem Mikromischer die partikelbildende flüssige Phase und das Dispersionsmedium zugeführt.

Die partikelbildende flüssige Phase kann sein:
1. die wäßrige Lösung einer partikelbildenden Substanz (z.B. Gelatinelösung, Wirkstofflösung)
2. eine organische Lösung einer partikelbildenden Substanz (z.B. PLGA in Ethylacetat, Wirkstofflösung, Wirkstoffsuspension)
3. eine geschmolzene (z.B. Fett, Wirkstoff, Polymer) partikelbildende Substanz

Der partikelbildenden flüssigen Phase können ggf. Tenside, Viskositätserhöher, Lösungsmittel oder Stabilisatoren zugesetzt werden.

Das Dispersionsmedium kann sein:
1. Wasser oder eine wäßrige Lösung
2. eine hydrophile Flüssigkeit (z.B. Glycerol)
3. eine organische Lösung
4. ölige Flüssigkeit (z.B. mittelkettige Trigylceride, Rizinusöl, Erdnußöl)
5. ein- oder mehrphasige Mischungen aus den Medien 1 bis 4

Dem Dispersionsmittel können die Koazervation auslösende oder Iöslichkeitsvermindernde Substanzen, Tenside, Viskositätserhöher und/oder Lösungsmittel zugesetzt werden.

Die Mikropartikel- oder Nanopartikelbildung erfolgt je nach partikelbildender flüssiger Phase durch Erstarrung, Verfestigung durch Lösungsmittelentzug und/oder Koazervation.

Im folgenden werden die verschiedenen möglichen Verfahrensvarianten näher beschrieben:

### Verfahrensvariante I:

Die partikelbildende Substanz (z.B. synthetisches Polymer, natürliche Substanz, Wirkstoff) wird in einer organischen Phase gelöst und diese dann als innere Phase (I) mit einer ihr nicht mischbaren äußeren Phase (A) mittels Mikromischer dispergiert. Zur Stabilisierung der erhaltenen Dispersion können den Phasen Tenside, Viskositätserhöher, Lösungsmittel oder andere Stabilisatoren zugesetzt sein. Die Entfernung des Lösungsmittels erfolgt durch kontinuierliche Evaporation über einen Dünnschichtverdampfer (z.B. Sambay) oder durch cross-flow-Filtration. Zur Verfestigung der Partikel kann eine separate Entfernung des Lösungsmittels entfallen, wenn das Lösungsmittel eine ausreichend hohe Löslichkeit in Wasser besitzt. Die Partikel können in diesem Fall durch konventionelle Trennmethoden wie Filtration, Sedimentation oder Zentrifugation abgetrennt werden.

### Verfahrensvariante II

Die partikelbildende Substanz (z.B. synthetisches Polymer, natürliche Substanz, Wirkstoff) wird in einer organischen Phase gelöst und dann als innere Phase (I) mit einer ihr mischbaren äußeren Phase (A) mittels Mikromischer dispergiert. Das Lösungsmittel der inneren Phase diffundiert in das Lösungsmittel der äußeren Phase und die partikelbildende Substanz bildet Partikel aus.
Zur Stabilisierung der erhaltenen Dispersion können den Phasen Tenside, Viskositätserhöher, weitere Lösungsmittel und/oder andere Stabilisatoren zugesetzt sein.
Die Entfernung des Lösungsmittels erfolgt durch kontinuierliche Evaporation über einen Dünnschichtverdampfer (z.B. Sambay) oder durch cross-flow-Filtration. Die Partikel können auch durch konventionelle Trennmethoden wie Filtration, Sedimentation oder Zentrifugation abgetrennt werden.

### Verfahrensvariante III:

Alternativ zur Partikelbildung durch Lösungsmittelentzug können die Partikel auch durch Koazervation hergestellt werden. Dabei ist die partikelbildende Substanz in dem Dispersionsmedium, das auch Stabilisatoren enthalten kann, gelöst. Im Mikromischer wird nun eine ganz oder teilweise im Dispersionsmedium unlösliche innere Phase, die wirksoffhaltig sein kann, mit dem Dispersionsmedium dispergiert. Die Koazervation kann durch physikalische Maßnahmen wie die Veränderung der Temperatur oder des pH-Wertes, sowie durch den Zusatz von Salzen (z.B. Natriumsulfat) oder Alkohol ausgelöst werden, wobei sich die gelöste partikelbildende Substanz auf der dispersen Phase abscheidet (einfache Koazervation).
Eine die Koazervation auslösende Substanz kann im Dispersionsmedium oder einer Vorlage gelöst sein, in die die Dispersion eingeleitet wird.

### Verfahrensvariante IV:

Partikel können aber auch ohne Lösungsmittel hergestellt werden, wenn die partikelbildende Substanz durch Erwärmen in den flüssigen Aggregatzustand überführt werden kann (z.B. bei Raumtemperatur feste Lipide, Wachse, Polymere oder Wirkstoffe). Man verfährt in diesem Fall in der Weise, daß in dem Mikromischer eine geschmolzene partikelbildende Substanz in einem geeigneten Dispersionsmedium bei erhöhter Temperatur dispergiert wird. In dem Dispersionsmedium können gegebenenfalls noch Tenside gelöst sein.

### Verfahrensvariante V:

Zur Herstellung von Kapseln kann z.B. ein flüssiger Kapselinhalt (Öl oder flüssiges Lipid) im Mikromischer in einer äußeren Phase dispergiert werden, die die partikelbildende Substanz gelöst enthält (z.B. Gelatine in Wasser). Die hergestellte Dispersion wird direkt in eine Fällungslösung geleitet (z.B. 5 % NaCl-Lösung). Die partikelbildende Substanz (z.B.Gelatine) fällt aus und zieht sich im Rahmen des Phasenseparationsprozesses unter Bildung der Kapselwand auf den Kapselinhalt auf.

### Verfahrensvariante VI:

Zur Herstellung von Kapseln nach dem W₁/O/W₂-Prinzip werden zwei Mikromischer oder ein statischer Mischer und ein Mikromischer hintereinandergeschaltet. Im ersten Mischer wird die W₁ Phase im organischen Lösungsmittel dispergiert, das die partikelbildende Substanz in gelöster Form enthält (O Phase), z.B. PLA in Ethylacetat. Im nachgeschalteten Mischer wird dann die W₁/O Emulsiori in der wäßrigen Phase (W₂) dispergiert. Es entsteht ein W₁/O/W₂-System, das wie unter Variante I getrocknet werden kann.
Den Phasen können ggf. Salze, Tenside oder Lösungsmittel zugesetzt sein.

### Verfahrensvariante VII:

Eine Mikropartikelbildung kann auch durch Vernetzung von gelöster partikelbildender Substanz (vorwiegend natürliche Polymere) erfolgen. Die Vernetzung zu unlöslichen Verbindungen kann durch geeignete chemische Reaktionen (Vernetzung von Proteinen mit Aldehyden) oder aber auch durch entgegengesetzt geladene lonen (z.B. durch Ca²⁺ bei Alginaten) herbeigeführt werden. Man verfährt in diesem Fall beispielsweise so, daß in dem Mikromischer eine Phase, in der die partikelbildende Substanz, sowie das beispielsweise durch Komplexierung inaktivierte Vemetzungsreagenz gelöst sind, mit einer zweiten, den Stabilisator (z.B. Tensid) enthaltenden Phase, dispergiert wird. Der Aktivator für das Vernetzungsreagenz kann sowohl im Dispersionsmittel als auch in einer Vorlage, in der die hergestellte Dispersion aufgefangen wird, enthalten sein. Der Aktivator kann beispielsweise eine Substanz sein, die die Freisetzung eines komplexierten Vernetzungsreagenzes aus dem Komplex bewirkt.

Das Phasenvolumenverhältnis I:A (innere Phase (I) zu äußerer Phase (A)) beträgt 1:1 bis 1:20, vorzugsweise 1:2 bis 1:10.
Bei einer Partikelherstellung über Doppelemulsionen vom Typ W₁/O/W₂ beträgt das Phasenvolumenverhältnis W₁:O:W₂ 1:1:1 bis 1:10:100, vorzugsweise 1:2:4 bis 1:8:50.

Als partikelbildende Substanz können bioabbaubare, synthetische und/oder natürliche Substanzen eingesetzt werden. Die Partikel können auch aus reinem Wirkstoff bestehen. Die partikelbildende Substanz kann vorgefertigt sein oder während der Herstellung z.B. durch Polymerisation entstehen.

Als bioabbaubare synthetische Polymere sind bevorzugt Polyester von Hydroxycarbonsäuren, die in dem erfindungsgemäßen Verfahren eingesetzt werden können:
Polyglycolide (PGA)und Copolymere von Glycoliden wie Glycolid/Lactid Copolymere (PGA/PLA=PLGA) oder Glycolid/Trimethylencarbonat Copolymere (PGA/TMC); L-Polylactide (PLA) und Stereocopolymere von Polylactiden wie Poly-L-Lactid (PLLA), Poly-DL-Lactid Copolymere und L-Lactid /DL-Lactid Copolymere; Copolymere von PLA wie Lactid/Tetramethylglycolid Copolymere, Lactid/δ-Valerolacton Copolymer und Lactid/ε-Caprolacton Copolymer; Poly-β-hydroxybutyrat (PHBA), PHBA/β-Hydroxyvalerat Copolymere (PHBA/HVA), Poly-β-hydroxypropionat (PHPA), Poly-p-dioxanon (PDS), Poly-δ-valerolacton, hydrophobisierte Polysaccharide, - Hyaluronsäure, - Dextrane oder hydrophobisiertes Amylopektin und Poly-ε-caprolacton.

Als Blockcopolymere von Polyestern von Hydroxycarbonsäuren und linear- oder Star-Polyethylenglykol (PEG) können in dem erfindungsgemäßen Verfahren die nachfolgend genannten Anwendung finden:
AB-Blockcopolymere aus PLA oder PLGA und PEG, ABA-Triblock-Copolymere aus PLA-PEG-PLA bzw. -PLGA, S(3)-PEG-PLA bzw. -PLGA Blockcopolymere und S(4)-PEG-PLA bzw. -PLGA Blockcopolymere.

Bevorzugt sind gemäß der Erfindung PLGA-Polymere mit einem Lactid/Glycolidverhältnis von 50:50, 75:25, 85:15 oder dazwischen liegende Mischungen. Die verwendeten mittleren Molekulargewichte liegen zwischen 1000 und 300000 Dalton. Es können auch Mischungen verschiedener Molekulargewichte vorliegen. Bevorzugt sind mittlere Molekulargewichte zwischen 10000 und 250000 Dalton.
Beispiele für diese bevorzugten Polymere sind Resomer®-RG-505 insbesondere Resomer® RG-752, Resomer® RG-756 oder Resomer® RG- 858.

Daneben können auch Polyamide (z.B. Poly-ε-caprolactam), Polyanhydride (z.B. Poly-carboxyphenoxyalkylcarbonsäuren), Polyorthoester, Polyacetate, Polyorthocarbonate, Polyphosphazene u. a. geeignete Polymere Verwendung finden.

Bevorzugte erfindungsgemäße natürliche Substanzen sind Fette (Lipide und Lipoide), natürliche und künstliche Mono-, Di- und Triglyceride, natürliche und künstliche Wachse, Kohlenwasserstoffe, Fettalkohole und ihre Ester und Ether, Lipidpeptide, Proteine und Zucker, sowie deren Derivate und Gemische wie z.B.:
Glycerintrilaurat, -myristat, -palmitat, -stearat, -behenat, Glycerintrioleat, Glycerolmonopalmitostearat, Cetylpalmitat, Kokosfett, Stearylalkohol-, Glycol-, Butandiol- und Glycerolester folgender Fettsäuren:
   Ameisen-, Essig-, Propion-, Butter-, Valerian-, Capron-, Önanth-, Capryl-, Pelargon-, Caprin-, Undecan-, Laurin-, Tridecan-, Myristin-, Pentadecan-, Palmitin-, Margarin-, Stearin-, Nonadecan-, Arachin-, Behen-, Lignocerin-, Cerotin-, Melissin-, Isobutter-, Isovalerian-, Tuberculostearin-,Acryl-, Croton-, Palmitolein-, Öl- ,Eruca-, Sorbin-, Linol-, Linolen-, Elaeostearin-, Arachidon-, Clupanodon- und/oder Docosahexaensäure.
   Hartparaffin, Oleylalkohol, Stearylalkohol, Cetylalkohol, gebleichtes Wachs, Gelatine, Humanserumalbumin, Bovinserumalbumin, Natriumalginat, Chitosan, Cellulose, Methylcellulose, Ethylcellulose, Hydroxypropylcellulose, Hydroxyethylcellulose, Natriumcarboxymethylcellulose, Pektin, Xanthan und
   Stärke oder deren Gemische.

Erfindungsgemäß einsetzbare Lösungsmittel oder Lösungsmittelgemische sind Ethanol, Isopropanol, Methanol, Alkylacetate wie Methyl-, Ethyl-, Propyl-, Isopropyl- oder Butylacetat, Alkylformiate wie Methyl-, Ethyl-, Propyl-, Isopropyloder Butylformiat, Triacetin, Triethylcitrat und/oder C₁-C₄ -Alkyllactate z. B. Methyl- oder Ethyllactat, Ketone z.B. Aceton, Ethylmethylketon. Bevorzugt werden Methylacetat, Ethylacetat, Propylacetat, Isopropylacetat und Propylformiat eingesetzt.

Im Sinne der Erfindung werden als oberflächenaktive Substanzen Poloxamere (Polyoxyethylen-Polyoxypropylen Blockcopolymere), Poloxamine (Polyoxyethylen-Polyoxypropylen Blockpolymere des Ethylendiamins), Polyethylenglycol Alkylether, Sorbitanfettsäureester (Span®), ethoxylierte Sorbitanfettsäureester (Polysorbate, Tween®), Saccharosefettsäureester, Gelatine, Polyvinylpyrrolidon, Fettalkoholpolyglycosid, CHAPS, CHAPSO, Decyl-β-D-Glycopyranosid, Decyl-β-D-Maltopyranosid, Dodecyl-β-D-Maltopyranosid, Natriumoleat, Polyethylenglykol, Polyvinyalkohol, polyoxyethylierte Fettsäureether (Brij®), Alkylphenolpoly(oxy)ethylene (z.B. Triton X-100®), Phospholipide wie z.B. Lecithine oder Lecithinderivate, Cholesterin und Cholesterinderivate, Cholate oder deren Gemische.

Bevorzugt finden Phospholipide, Polyvinylalkohol, polyoxyethylierte Fettsäureether (Brij®), Poloxamere (Polyoxyethylen-Polyoxypropylen Blockcopolymere), Poloxamine (Polyoxyethylen-Polyoxypropylen Blockpolymere des Ethylendiamins), ethoxylierte Sorbitanfettsäureester (Polysorbate, Tween®), Natriumcholat und Saccharosefettsäureester oder deren Gemische Anwendung.

Weiterhin ist die Verwendung viskositätserhöhender Substanzen zur Stabilisierung der inneren und äußeren Phase möglich.
Es können beispielsweise Celluloseether und -ester wie Methylcellulose, Hydroxyethylcellulose oder Hydroxypropylcellulose, Polyvinylderivate wie Polyvinylalkohol, Polyvinylpyrrolidon oder Polyvinylacetat, Polyacrylsäure (z.B. Carbopol) und deren Derivate sowie Xanthane oder Pektine und deren Gemische eingesetzt werden.

Gegenstand der Erfindung sind auch morphologisch einheitliche Mikro- bzw. Nanopartikelpartikel, die nach dem genannten Verfahren hergestellt werden. Die Partikelgrößenverteilungen sind sehr eng.

Anschließend wird die Erfindung an Ausführungsbeispielen näher erläutert, ohne sie darauf einzuschränken.

### Beispiel 1: Herstellung von PLA-Mikropartikeln

### Verfahrensvariante I

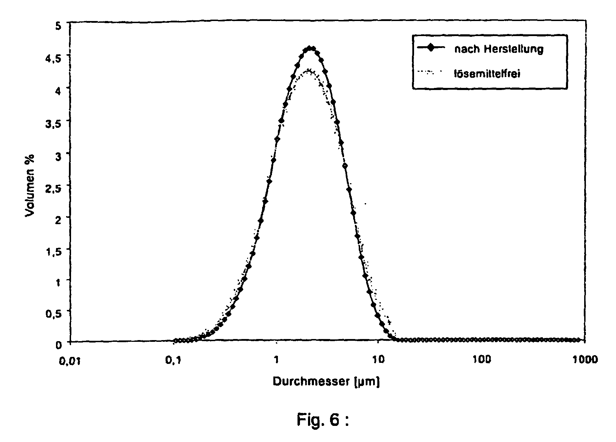

2,0 g Resomer RG 858 werden in 38,0 g Ethylacetat gelöst (innere Phase = I). 2,0 g Poloxamer 188 (Synperonic F68) werden in 198,0 g destilliertem Wasser gelöst (äußere Phase = A). Die Lösungen werden mittels HPLC Pumpen (Gynkotec) durch einen Mikromischer (Institut für Mikrotechnik, Mainz (IMM), Deutschland) gepumpt. Die Mikrokanalbreite beträgt 40 µm. Das Phasenvolumenverhältnis I:A beträgt 1:8. Die Partikelgrößenbestimmung mittels Laserdiffraktometrie (LS 130, Coulter Electronics, USA) der frisch dispergierten Partikel ergibt einen mittleren Volumen-Durchmesser D₅₀ von 3,0 µm, 98 % der Partikel liegen zwischen 0,5 µm und 11,1 µm. Der mittlere Durchmesser D₅₀ der Anzahlverteilung beträgt 606 nm.
Die Vollständigkeit der Lösungsmittelentfemung kann dadurch gezeigt werden, daß auch eine Nachtrocknung nicht mehr zu einer Abnahme der Partikelgröße führt. Die Größe der Partikel nach Dispergierung mit dem Mischer und nach Nachtrocknung sind praktisch identisch (Fig. 6).

### Beispiel 2: Herstellung von Partikeln aus Hartfett (Witepsol H5):

### Verfahrensvariante IV

40,0 g Hartfett (Witepsol H5) werden bei 70 °C aufgeschmolzen (innere Phase = I). 2,0 g Poloxamer 188 (Synperonic F68) werden in 198,0 g destilliertem Wasser gelöst (äußere Phase = A). Die äußere Phase wird ebenfalls auf 70 °C erhitzt. Die Lösungen werden mittels HPLC Pumpen (Gynkotec) durch einen Mikromischer (IMM) gepumpt. Die Mikrokanalbreite beträgt 40 µm. Das Phasenvolumenverhältnis I:A beträgt 1:7.
Die Partikelgrößenbestimmung mittels Laserdiffraktometrie ergibt einen mittleren Volumen-Durchmesser D₅₀ von 2,4 µm, 98 % der Partikel liegen zwischen 0,6 µm und 6,9 µm.

### Beispiel 3: Gelatinekapseln

### Verfahrensvariante III

1,0 g Gelatine werden in 39,0 g destilliertem Wasser bei 70 °C gelöst (äußere Phase = A). 10 ml Maiskeimöl werden auf 70 °C erwärmt (innere Phase = I). Die Lösungen werden mittels HPLC Pumpen durch einen Mikromischer (IMM) gepumpt. Die Mikrokanalbreite beträgt 40 µm. Das Phasenvolumenverhältnis I:A beträgt 1:7. Die Partikelgrößenbestimmung mittels Laserdiffraktometrie ergibt einen mittleren Volumen-Durchmesser D₅₀ von 2,3 µm, 98 % der Partikel liegen zwischen 0,4 µm und 9,1 µm.

### Beispiel 4 Alginatpartikel, Einheitlichkeit der Partikel

### Verfahrensvariante VII

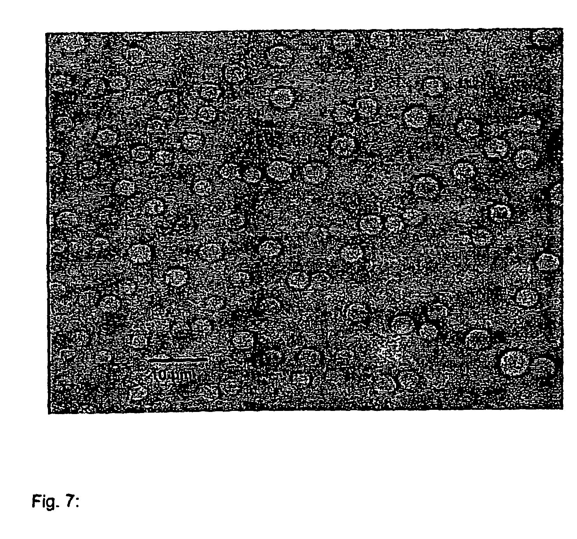

0,5 g Natriumalginat und 0,25 g CaCl₂x2H₂O werden in 50,0 ml wäßriger 0,1 M EDTA-Lösung gelöst und mit destilliertem Wasser auf 160,0 ml ergänzt (innere Phase ). 1,0 g Span 80 werden in 175,0 g Propylacetat gelöst (äußere Phase = A). Als Vorlage diente eine Lösung von Acetanhydrid in Propylacetat. Die Lösungen werden mittels HPLC Pumpen (Gynkotec) durch einen Mikromischer (Institut für Mikrotechnik. Mainz, Deutschland) in die Vorlage gepumpt. Die Mikrokanalbreite beträgt 40 µm. Das Phasenvolumenverhältnis I:A beträgt 1:3. Die einheitliche Größe der erhaltenen Mikropartikel zeigt das mikroskopische Bild in Fig. 7.

### Beispiel 5: Einheitlichkeit des Produktes nach Herstellung mit Mikromischer

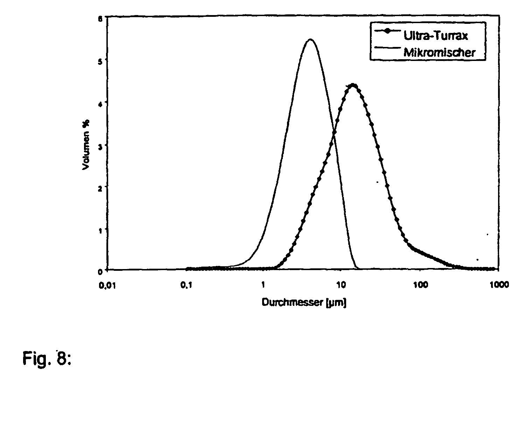

PLA-Mikropartikel werden nach Beispiel 1 hergestellt. Zum Vergleich erfolgt die Herstellung mit einem hochtourigen Rührer Ultra-Turrax (Janke und Kunkel, Modell RW20 DZM) unter Verwendung des Rührwerkzeugs S 25N-10G bei 24000 Umdrehungen pro Minute für 5 Minuten. Fig. 8 zeigt die mit beiden Methoden erhaltenen Partiketgrößenverteilungen. Die erfindungsgemäß hergestellten Partikel sind hinsichtlich der Einheitlichkeit den Partikeln, die mittels Ultra-Turrax hergestellt werden, eindeutig überlegen.

### Beispiel 6: Reproduzierbarkeit der Herstellmethode:

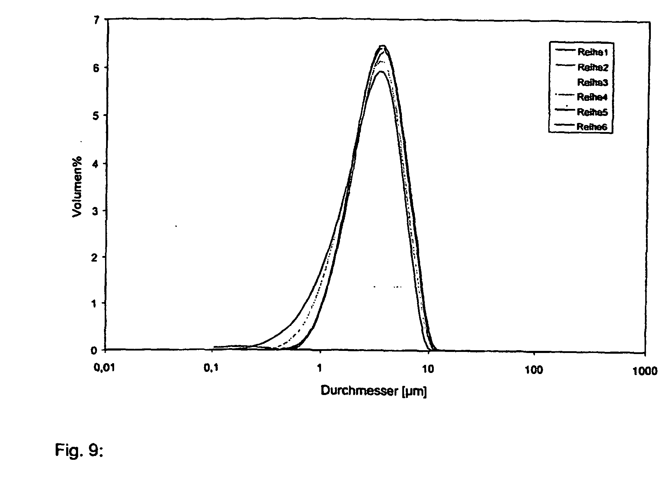

Die Rezeptur von Beispiel 1 wird sechsmal hergestellt und die Partikelgröße mittels Laserdiffraktometrie analysiert. Fig. 9 zeigt ein Overlay sämtlicher 6 Kurven, Tab. 1 gibt die wichtigsten Durchmesser an.

**Tab. 1:**

| **Volumen** % | **Reihe1** Partikel-Durchmesser | **Reihe2** Partikel-Durchmesser | **Reihe3** Partikel-Durchmesser | **Reihe4** Partikel-Durchmesser | **Reihe5** Partikel-Durchmesser | **Reihe6** Partikel-Durchmesser |
|---|---|---|---|---|---|---|
| | µm < | µm < | µm < | µm < | µm < | µm < |
| 1 | 0,856 | 0,571 | 0,707 | 0,634 | 0,393 | 0,890 |
| 10 | 1,525 | 1,492 | 1,324 | 1,279 | 1,031 | 1,543 |
| 25 | 2,203 | 2,211 | 1,961 | 1,958 | 1,74 | 2,211 |
| 50 | 3,278 | 3,328 | 2,952 | 3,028 | 2,835 | 3,275 |
| 75 | 4,742 | 4,826 | 4,236 | 4,428 | 4,188 | 4,716 |
| 90 | 6,379 | 6,456 | 5,612 | 5,941 | 5,596 | 6,297 |
| 99 | 9,235 | 9,164 | 7,965 | 8,595 | 7,944 | 8,892 |
| Standardabweichung s [µm] | 1,91 | 1,94 | 1,67 | 1,82 | 1,76 | 1,85 |

### Beispiel 7: Testosteron Mikropartikel

### Verfahrensvariante II

0,2 g Testosteron werden in 9,8 g Aceton (innere Phase = I) gelöst. 1,4 g Synperonic®F68 in 68,6 g destilliertem Wasser gelöst (äußere Phase = A). Die Lösungen werden mittels HPLC Pumpen durch einen Mikromischer (IMM) gepumpt. Die Mikrokanalbreite beträgt 40 µm. Das Phasenvolumenverhältnis I:A beträgt 1:7. Die Partikelgrößenbestimmung mittels Laserdiffraktometrie ergibt einen mittleren Volumen-Durchmesser D₅₀ von 1,5 µm, 98 % der Partikel liegen zwischen 0,1 µm und 2,7 µm.

### Beispiel 8: Herstellung von methylenblauhaltigen PLGA Mikrokapseln

### Verfahrensvariante VI

2,0 g Resomer RG 858 und 0,4 g Span 80 werden in 38,0 g Ethylacetat gelöst (Phase O). 0,025 g Methylenblau werden in 9,975 g destilliertem Wasser gelöst (Phase W₁). Die Lösungen werden mittels HPLC Pumpen (Gynkotec) durch einen Mikromischer (Institut für Mikrotechnik, Mainz, Deutschland) gepumpt. Die Mikrokanalbreite beträgt 25 µm. Das Phasenvolumenverhältnis W₁:O beträgt 1:4.
Die entstandene W/O-Emusion wird in einen zweiten Mikromischer mit einer Mikrokanalbreite von 40 µm geleitet. Als zweite Phase wird eine wäßrige 4 %ige Poloxamer 188 (Synperonic F68) Lösung hinzugegeben (Phase W₂). Das Phasenvolumenverhältnis W₁:O:W₂ beträgt = 1:4:15.
Die resultierenden Kapseln zeigen einen mittleren Volumendurchmesser von D₅₀= 1,06 µm, 98 % der Mikrokapseln liegen zwischen 0,13 µm und 5,14 µm.

### Beispiel 9: Herstellung von Ethinylestradiol-haltigen PLA-Mikropartikeln

### Verfahrensvariante I

Es werden Mikropartikel analog Beispiel 1 hergestellt. Zusätzlich sind 0,2 g Ethinylestradiol in der organischen Phase gelöst.

### Beispiel 10: Herstellung von Estradiol-haltigen PLA-Mikropartikeln

### Verfahrensvariante I

Es werden Mikropartikel analog Beispiel 1 hergestellt. Zusätzlich sind 0,2 g Estradiol in der organischen Phase gelöst.

### Beispiel 11: Herstellung von Testosteron-haltigen PLA-Mikropartikeln

### Verfahrensvariante I

Es werden Mikropartikel analog Beispiel 1 hergestellt. Zusätzlich sind 0,2 g Testosteron in der organischen Phase gelöst.

### Beispiel 12: Herstellung von Gestoden-haltigen PLA-Mikropartikeln

### Verfahrensvariante I

Es werden Mikropartikel analog Beispiel 1 hergestellt. Zusätzlich sind 0,2 g Gestoden in der organischen Phase gelöst.

### Beispiel 13: Herstellung von Levonorgestrel-haltigen PLA-Mikropartikeln

### Verfahrensvariante I

Es werden Mikropartikel analog Beispiel 1 hergestellt. Zusätzlich sind 0,2 g Levonorgestrel in der organischen Phase gelöst.

### Beispiel 14: Herstellung von Poloxamer® F68-stabilisierten, Humanserumalbumin-haltigen PLGA-Mikrokapseln

### Verfahrensvariante VI

5,0 g Resomer RG 752 werden in 95,0 g Ethylacetat gelöst (Phase O). 0,200 g Humanserumalbumin (HSA) werden in 19,8 g destilliertem Wasser gelöst (W₁- Phase). Die Lösungen werden mittels HPLC Pumpen (Gynkotec) durch einen Mikromischer (Institut für Mikrotechnik, Mainz, Deutschland) gepumpt. Die Mikrokanalbreite beträgt 40 µm. Das Phasenvolumenverhältnis W₁:O beträgt 1:6.
Die entstandene W₁/O-Emusion wird in einen zweiten Mikromischer mit einer Mikrokanalbreite von 40 µm geleitet. Als zweite Phase wird eine wäßrige 4 %ige Poloxamer 188 (Synperonic F68) Lösung hinzugegeben (Phase W₂). Das Phasenvolumenverhältnis W₁:O:W₂ beträgt 1:6:21
Die resultierenden Kapseln zeigen einen mittleren Volumendurchmesser von D₅₀= 1,06 µm, 90 % der Mikrokapseln sind kleiner 1,80 µm.
Die Beladung der Mikrokapseln betrug 6,7% der theoretisch zu erzielenden Beladung von 3,23%(m/m), d.h. es wurde eine absolute Beladung von 0,22% (m/m) erreicht.

### Beispiel 15: Herstellung von Natriumcholat stabilisierten, Humanserumalbumin-haltigen PLGA-Mikrokapseln

### Verfahrensvariante VI

Es werden Mikrokapseln analog Beispiel 14 hergestellt, wobei in der äußeren wäßrigen (W₂-) Phase anstelle des Poloxamer F68 nun 0,5 % (m/m) Natriumcholat zur Stabilisierung verwendet wird. Der mittlere Volumendurchmesser D₅₀ beträgt 2,18 µm, der D₉₀ beträgt 4,15 µm.

### Beispiel 14: Herstellung von Poloxamer® stabilisierten, Humanserumalbuminhaltigen PLGA-Mikrokapseln, Ethanolzusatz

### Verfahrensvariante VI

Es werden Mikrokapseln analog Beispiel 14 hergestellt, wobei die äußere wäßrige Phase neben 4 % (m/m) Poloxamer® noch einen Anteil von 37,5 % (m/m) Ethanol enthielt. Der mittlere Volumendurchmesser D₅₀ beträgt 1,15 µm, der D₉₀ beträgt 3,13 µm.

## Patentansprüche

1. Kontinuierliches Verfahren zur Herstellung von Mikro- oder Nanopartikeln aus bioabbaubaren synthetischen und/oder natürlichen Substanzen, **dadurch gekennzeichnet, dass** die Herstellung mittels eines Mikromischers erfolgt, der eine Mischkammer mit ineinandergreifenden Mikrokanälen enthält, in der Flüssigkeitslamellen der zu mischenden Flüssigkeiten periodisch zur Überlappung gebracht werden.

2. Verfahren gemäß Anspruch 1, **dadurch gekennzeichnet, dass** die zu mischenden Flüssigkeiten eine partikelbildende flüssige Phase und ein Dispersionsmittel ist.

3. Verfahren nach Anspruch 2, **dadurch gekennzeichnet, dass** die partikelbildende flüssige Phase eine wäßrige oder organische Lösung einer partikelbildenden Substanz oder eine geschmolzene partikelbildende Substanz ist, der ggf. Tenside, Viskositätserhöher oder andere Stabilisatoren zugesetzt werden.

4. Verfahren nach Anspruch 2, **dadurch gekennzeichnet, dass** das Dispersionsmedium Wasser, eine wäßrige Lösung, eine hydrophile Flüssigkeit, eine organische Lösung oder eine ölige Flüssigkeit ist, der ggf. eine die Koazervation auslösende und/oder Iöslichkeitsvermindernde Substanz, Tenside und/oder Viskositätserhöher zugesetzt werden.

5. Verfahren nach Anspruch 2, **dadurch gekennzeichnet, dass** das Dispersionsmedium eine ein- oder mehrphasige Mischung aus mindestens zwei Dispersionsmedien ist.

6. Verfahren nach einem der Ansprüche 1 bis 5, **dadurch gekennzeichnet, dass** die Mikrokanäle eine Mikrokanalbreite von 1 bis 1000 µm aufweisen.

7. Verfahren nach einem der Ansprüche 1 bis 6, **dadurch gekennzeichnet, dass** die Wände der Mikrokanäle wellenförmig, parallel oder zickzackförmig ausgestaltet sind.

8. Verfahren nach einem der Ansprüche 1 bis 7, **dadurch gekennzeichnet, dass** die Mischkammer eine LIGA-Mischkammer ist.

9. Verfahren nach einem der Ansprüche 1 bis 8, **dadurch gekennzeichnet, dass** die partikelbildende flüssige Phase und das Dispersionsmedium dem Mikromischer im Gegenfluß, Gleichfluß oder koaxial zugeführt werden.

10. Verfahren nach einem der Ansprüche 1 bis 9, **dadurch gekennzeichnet, dass** die partikelbildende flüssige Phase und das Dispersionsmedium dem Mikromischer drucklos oder bei Flüssigkeitsdrücken von bis zu ca. 30 bar zugeführt werden.

11. Verfahren nach einem der Ansprüche 1 bis 10, **dadurch gekennzeichnet, dass** das Mischprodukt senkrecht oder parallel zur Flußrichtung gewonnen wird.

12. Verfahren nach einem der Ansprüche 1 bis 11, **dadurch gekennzeichnet, dass** das Verfahren zur Verkapselung von Wirkstoffen verwendet wird.

13. Verfahren nach einem der Ansprüche 1 bis 12, **dadurch gekennzeichnet, dass** das Verfahren zur Herstellung von Wirkstoffpartikeln verwendet wird.

14. Verfahren nach einem der Ansprüche 1 bis 13,**dadurch gekennzeichnet, dass** der zu verkapselnde Wirkstoff, direkt oder gelöst in einem geeigneten Medium, in der partikelbildenden flüssigen Phase gelöst, suspendiert oder emulgiert wird.

15. Verfahren nach einem der Ansprüche 1 bis 14, **dadurch gekennzeichnet, dass** die Emulgierung eines Wirkstoffes oder einer Wirkstofflösung in die flüssige partikelbildende Phase durch einen vorgeschalteten Mischer, vorzugsweise Mikromischer erfolgt.

16. Verfahren nach einem der Ansprüche 1 bis 15, **dadurch gekennzeichnet, dass** der oder die Wirkstoffe oder die Wirkstofflösung durch eine weitere Einlaßbohrung dem Mikromischer zugeführt wird und die Vermischung im Mikromischer erfolgt.

17. Verfahren nach einem der Ansprüche 1 bis 16, **dadurch gekennzeichnet, dass** als Lösungsmittel Ethanol, Isopropanol, Methanol, Alkylacetate wie Methyl-, Ethyl-, Propyl-, Isopropyl- oder Butylacetat, Alkylformiate wie Methyl-, Ethyl-, Propyl-, Isopropyl- oder Butylformiat, Triacetin, Triethylcitrat und/oder C₁-C₄-Alkyllactate z.B. Methyl- oder Ethyllactat, Ketone z.B. Aceton, Ethylmethylketon verwendet werden.

18. Verfahren nach einem der Ansprüche 1 bis 17, **dadurch gekennzeichnet, dass** als Lösungsmittel Methylacetat, Ethylacetat, Propylacetat, Isopropylacetat und Propylformiat verwendet werden.

19. Verfahren nach einem der Ansprüche 1 bis 18, **dadurch gekennzeichnet, dass** als bioabbaubare synthetische vorgefertigte Polymere Polyester von Hydroxycarbonsäuren eingesetzt werden: Polyglycolide (PGA) und Copolymere von Glycoliden wie Glycolid/Lactid Copolymere (PGA/PLLA=PLGA) oder Glycolid/Trimethylencarbonat Copolymere (PGA/TMC);L- Polylactide (PLA) und Stereocopolymere von Polylactiden wie Poly-L-Lactid (PLLA), Poly-DL-Lactid Copolymere und L-Lactid/DL-Lactid Copolymere; Copolymere von PLA wie Lactid/Tetramethylglycolid Copolymere, Lactid/δ-Valerolacton Copolymer und Lactid/ε-Caprolacton Copolymer; Poly-β-hydroxybutyrat (PHBA), PHBA/β-Hydroxyvalerat Copolymere (PHBA/HVA), Poly-β-hydroxypropionat (PHPA), Poly-ρ-dioxanon (PDS), Poly-δ-valerolacton, hydrophobisierte Polysaccharide, - Hyaluronsäure, - Dextrane oder hydrophobisiertes Amylopektin und Poly-ε-caprolacton.

20. Verfahren nach einem der Ansprüche 1 bis 19, **dadurch gekennzeichnet, dass** als bioabbaubare synthetische Polymere Blockpolymere von Polyestern von Hydroxycarbonsäuren und linear- oder Star-Polyethylenglykol (PEG) eingesetzt werden: AB-Blockcopolymere aus PLA oder PLGA und PEG, ABA-Triblock-Copolymere aus PLA-PEG-PLA bzw. -PLGA, S(3)-PEG-PLA bzw. -PLGA Blockcopolymere und S(4)-PEG-PLA bzw. -PLGA Blockcopolymere.

21. Verfahren nach einem der Ansprüche 1 bis 20, **dadurch gekennzeichnet, dass** als bioabbaubare synthetische Polymere Glycolid/Lactid Copolymere wie Resomer ® RG-505, Resomer ® RG-756 oder Resomer ® RG-858 mit einem Lactid/Glycolidverhältnis von 50:50, 75:25, 85:15 oder dazwischen liegende Mischungen und einem mittleren Molekulargewicht zwischen 10000 und 250000 eingesetzt werden.

22. Verfahren nach einem der Ansprüche 1 bis 21, **dadurch gekennzeichnet, dass** als natürliche Substanzen Fette (Lipide und Lipoide), natürliche und künstliche Mono-, Di- und Triglyceride, natürliche und künstliche Wachse, Kohlenwasserstoffe, Fettalkohole und ihre Ester und Ether, Lipidpeptide, Proteine und Zucker, sowie deren Derivate und/oder deren Gemische verwendet werden.

23. Verfahren nach Anspruch 22, **dadurch gekennzeichnet, dass** als natürliche Substanzen verwendet werden:
Glycerintrilaurat, -myristat, -palmitat, -stearat, -behenat, Glycerintrioleat, Glycerolmonopalmitostearat, Cetylpalmitat, Kokosfett, Stearylalkohol-, Glycol-, Butandiol- und Glycerolester folgender Fettsäuren:
Ameisen-, Essig-, Propion-, Butter-, Valerian-, Capron-, Onanth-, Capryl-, Pelargon-, Caprin-, Undecan-, Laurin-, Tridecan-, Myristin-, Pentadecan-, Palmitin-, Margarin-, Stearin-, Nonadecan-, Arachin-, Behen-, Lignocerin-, Cerotin-, Melissin-, Isobutter-, Isovalerian-, Tuberculostearin-, Acryl-, Croton-, Palmitolein-, Öl-, Eruca-, Sorbin-, Linol-, Linolen-, Elaeostearin-, Arachidon-, Clupanodon- und/oder Docosahexaensäure Hartparaffin, Oleylalkohol, Stearylalkohol, Cetylalkohol, gebleichtes Wachs, Gelatine, Humanserumalbumin, Bovinserumalbumin, Natriumalginat, Chitosan, Cellulose, Methylcellulose, Ethylcellulose, Hydroxypropylcellulose, Natriumcarboxymethylcellulose, Pektin, Xanthan und Stärke oder deren Gemische.

24. Verfahren nach einem der Ansprüche 1 bis 23, **dadurch gekennzeichnet, dass** als oberflächenaktive Substanzen eingesetzt werden:
Poloxamere (Polyoxyethylen-Polyoxypropylen Blockcopolymere), Poloxamine (Polyoxyethylen-Polyoxypropylen Blockpolymere des Ethylendiamins), Polyethylenglycol Alkylether, Sorbitanfettsäureester (Span ®), ethoxylierte Sorbitanfettsäureester (Polysorbate, Tween ®), Saccharosefettsäureester, Gelatine, Polyvinylpyrrolidon, Fettalkoholpolyglycosid, CHAPS, CHAPSO, Decyl-β-D-Glycopyranosid, Decyl-β-D-Maltopyranosid, Dodecyl-β-D-Maltopyranosid, Natriumoleat, Polyethylenglykol, Polyvinylalkohol, polyoxyethylierte Fettsäureether (Brij ®), Alkylphenolpoly(oxyethylene (z.B. Triton X-100 ®), Phospholipide wie z.B. Lecithine oder Lecithinderivate, Cholesterin und Cholesterinderivate, Cholate oder deren Gemische.

25. Verfahren nach Anspruch 24, **dadurch gekennzeichnet, dass** als oberflächenaktive Substanzen eingesetzt werden:
Phospholipide, Polyvinylalkohol, polyoxyethylierte Fettsäureether (Brij ®), Poloxamere (Polyoxyethylen-Polyoxypropylen Blockcopolymere), Poloxamine (Polyoxyethylen-Polyoxypropylen Blockpolymere des Ethylendiamins), ethoxylierte Sorbitanfettsäureester (Polysorbate, Tween ®), Natriumcholat und Saccharosefettsäureester oder deren Gemische.

26. Verfahren nach einem der Ansprüche 1 bis 25, **dadurch gekennzeichnet, dass** die Entfernung des Lösungsmittels bzw. Lösungsmittelgemisches, sowie ggf. die Entfernung von unverkaspeltem Wirkstoff und/oder des/der Tenside in einer cross-flow-Filtration oder mit einem Dünnschichtverdampfer erfolgt.

27. Verfahren nach einem der Ansprüche 1 bis 26, **dadurch gekennzeichnet, dass** ein scalig-up vom Labor auf einen Produktionsmaßstab durch die Parallelschaltung von mehreren Mikromischem und/oder durch den Einsatz von Mischerarrays (numbering-up) durchgeführt wird.

## Revendications

1. Procédé continu en vue de la fabrication de micro- ou de nanoparticules à partir de substances synthétiques et/ou naturelles biodégradables, **caractérisé en ce que** la fabrication se fait dans un micromélangeur, qui contient une chambre de mélange ayant des microcanaux interconnectés les uns avec les autres, dans lesquels des lamelles de liquide sont mises en superposition d'une manière périodique avec les liquides à mélanger.

2. Procédé selon la revendication 1, **caractérisé en ce que** les liquides à mélanger sont constitués d'une phase fluide formant des particules et d'un agent de dispersion.

3. Procédé selon la revendication 2, **caractérisé en ce que** la phase fluide formant des particules est une solution aqueuse ou organique d'une substance formant des particules ou une substance fondue formant des particules, à laquelle l'on ajoute le cas échéant, des agents tensioactifs, des agents d'augmentation de la viscosité, ou d'autres agents de stabilisation.

4. Procédé selon la revendication 2, **caractérisé en ce que** le milieu de dispersion est l'eau, une solution aqueuse, un liquide hydrophile, une solution organique ou un fluide huileux, auquel, le cas échéant, l'on ajoute une substance déclenchant la coagulation et/ou réduisant la solubilité, des agents tensioactifs et/ou des agents d'augmentation de la viscosité.

5. Procédé selon la revendication 2, **caractérisé en ce que** le milieu de dispersion est un mélange à une ou plusieurs phases d'au moins deux milieux de dispersion.

6. Procédé selon l'une quelconque des revendications 1 à 5, **caractérisé en ce que** les microcanaux présentent une largeur de microcanal de 1 à 1000 µm.

7. Procédé selon l'une quelconque des revendications 1 à 6, **caractérisé en ce que** les parois des microcanaux sont réalisées sous une forme ondulée, parallèle ou à zig zag.

8. Procédé selon l'une quelconque des revendications 1 à 7, **caractérisé en ce que** la chambre de mélange est une chambre de mélange LIGA.

9. Procédé selon l'une quelconque des revendications 1 à 8, **caractérisé en ce que** la phase fluide formant des particules et le milieu de dispersion sont acheminés au micromélangeur, à contre-courant, à co-courant ou d'une manière coaxiale.

10. Procédé selon l'une quelconque des revendications 1 à 9, **caractérisé en ce que** la phase fluide formant des particules et le milieu de dispersion sont acheminés au micromélangeur sans pression ou à des pressions de liquide allant jusqu'à environ 30 bars.

11. Procédé selon l'une quelconque des revendications 1 à 10, **caractérisé en ce que** le produit de mélange est obtenu d'une manière perpendiculaire ou parallèle à la direction d'écoulement.

12. Procédé selon l'une quelconque des revendications 1 à 11, **caractérisé en ce que** l'on utilise le procédé en vue du capsulage des agents actifs.

13. Procédé selon l'une quelconque des revendications 1 à 12, **caractérisé en ce que** l'on utilise le procédé en vue de la fabrication de particules d'agents actifs.

14. Procédé selon l'une quelconque des revendications 1 à 13, **caractérisé en ce que** l'agent actif à capsuler est mis en solution, en suspension ou en émulsion dans la phase fluide formant des particules, directement, ou sous une forme dissoute dans un milieu approprié.

15. Procédé selon l'une quelconque des revendications 1 à 14, **caractérisé en ce que** l'émulsification d'un agent actif ou d'une solution d'agents actifs se fait dans la phase fluide formant des particules à l'aide d'une mélangeur intercalé en amont, de préférence, d'un micromélangeur.

16. Procédé selon l'une quelconque des revendications 1 à 15, **caractérisé en ce que** le ou les agents actifs ou la solution d'agents actifs est (sont) acheminé (e) (s) au micromélangeur grâce à un alésage d'admission supplémentaire et que le mélange se fait dans le micromélangeur.

17. Procédé selon l'une quelconque des revendications 1 à 16, **caractérisé en ce que** l'on utilise, en tant que solvant, l'éthanol, l'isopropanol, le méthanol, des acétates d'alkyle, comme l'acétate de méthyle, d'éthyle, de propyle, d'isopropyle ou de butyle, des formiates d'alkyle comme le formiate de méthyle, d'éthyle, de propyle, d'isopropyle ou de butyle, la triacétine, le citrate de triéthyle, et/ou les lactates de C₁-C₄-alkyle, par exemple, le lactate de méthyle ou d'éthyle, des cétones, par exemple, l'acétone, la éthylméthylcétone.

18. Procédé selon l'une quelconque des revendications 1 à 17, **caractérisé en ce que** l'on utilise, en tant que solvants, l'acétate de méthyle, l'acétate d'éthyle, l'acétate de propyle, l'acétate d'isopropyle et le formiate de propyle.

19. Procédé selon l'une quelconque des revendications 1 à 18, **caractérisé en ce que** l'on utilise, en tant que polymères apprêtés synthétiques biodégradables, des polyesters d'acides hydroxycarboxyliques :
des polyglycolides (PGA) et des copolymères de glycolides comme des copolymères glycolide / lactide (PGA/PLLA=PLGA) ou des copolymères glycolide/carbonate de triméthylène (PGA/TMCL) ; des L-polylactides (PLA) et des stéréocopolymères de polylactides comme le poly-L lactide (PLLA), des copolymères poly-DL-lactide et des copolymères L-lactide/DL-lactide ; des copolymères de PLA comme les copolymères lactide/tétraméthylglycolide, le copolymère lactide/δ-valérolactone et le copolymère lactide/ε-caprolactone ; le poly-β-hydroxybutyrate (PHBA) / les copolymères PHBA/β-hydroxyvalérate (PHBA/HVA), le poly-β-hydroxypropionate (PHPA), la poly-ρ-dioxanone (PDS), le poly-δ-valérolactone, les polysaccharides rendues hydrophobes, l'acide hyaluronique de polysaccharides rendues hydrophobes, des dextranes de polysaccharides rendues hydrophobes ou l'amylopectine rendue hydrophobe et le poly-ε-caprolactone.

20. Procédé selon l'une quelconque des revendications 1 à 19, **caractérisé en ce que** l'on utilise, en tant que polymères synthétiques biodégradables, des polymères séquencés de polyesters d'acides hydroxycarboxyliques et de polyéthylèneglycol (PEG) linéaire ou rigide :
des copolymères séquencés AB en PLA ou PLGA et PEG, des copolymères tri-séquencés ABA en PLA-PEG-PLA ou -PLGA, des copolymères séquencés S(3)-PEG-PLA ou -PLGA et -PEG-PLA ou -PLGA.

21. Procédé selon l'une quelconque des revendications 1 à 20, **caractérisé en ce que** l'on utilise, en tant que polymères synthétiques biodégradables, des copolymères glycolide/lactide comme les produits Resomer® RG-505, Resomer® RG-756 ou Resomer® RG-858 avec un rapport lactide/glycolide de 50:50, 75:25, 85:15 ou des mélanges se situant dans les intervalles de rapport et un poids moléculaire moyen compris entre 10000 et 250000.

22. Procédé selon l'une quelconque des revendications 1 à 21, **caractérisé en ce que** l'on utilise, en tant que substances naturelles (lipides et lipoïdes) des mono-, di- et tri-glycérides naturels et synthétiques, des cires naturelles et synthétiques, des hydrocarbures, des alcools gras et leurs esters et éthers, des lipidpeptides, des protéines et des sucres, tout comme leurs dérivés et/ou leurs mélanges.

23. Procédé selon la revendication 22, **caractérisé en ce que** l'on utilise en tant que substances naturelles :
le trilaurate de glycérine, le myristate de glycérine, le palmitate de glycérine, le stéarate de glycérine, le béhénate de glycérine, le trioléate de glycérine, le monopalmitostéarate de glycérol, le palmitate de cétyle, la graisse de coco, les esters de l'alcool stéarylique, du glycol, du butanediol et du glycérol des acides gras suivants :
acides formique, acétique, propionique, butyrique, valérianique, caproïque, oenanthylique, caprylique, pélargonique, caprique, undécanoïque, laurinique, tridécanoïque, myristinique, pentadécanoïque, palmitinique, margarinique, stéarinique, nonadécanoïque, arachinique, béhénique, lignocérinique, cérotinique, mélissinique, isobutyrique, isovalérianique, tuberculostéarinique, acrylique, crotonique, palmitoléinique, oléique, érucacique, sorbinique, linoléique, linolénique, élaéostéarinique, arachidonique, clupanodonique et/ou docosahexaènorigue, la paraffine dure, l'alcool oléylique, l'alcool stéarylique, l'alcool cétylique, la cire blanchie, la gélatine, la sérumalbumine humaine, la sérumalbumine bovine, l'alginate de sodium, le chitosane, la cellulose, la méthylcellulose, l'éthylcellulose, l'hydroxypropylcellulose, la carboxyméthylcellulose de sodium, la pectine, le xanthane et des amidons et leurs mélanges.

24. Procédé selon l'une quelconque des revendications 1 à 23, **caractérisé en ce que** l'on utilise en tant que substances tensioactives : des poloxamères (copolymères séquencés polyoxyéthylène polyoxypropylène, poloxamines (copolymères séquencés polyoxyéthylène-polyoxypropylène de l'éthylènediamine), des éthers alkyles du polyéthylèneglycol, l'ester d'acide gras de la sorbitane (Span®), l'ester éthoxylé d'acide gras de la sorbitane (polysorbate, Tween®), des esters d'acide gras de la saccharose, la gélatine, le polyvinylpyrrolidone, le polyglycoside d'alcool gras, le CHAPS, le CHAPSO, le décyl-β-D-glycopyranoside, le décyl-β-D-maltopyranoside, le dodécyl-β-D-maltopyranoside, l'oléate de sodium, le polyéthylèneglycol, l'alcool polyvinylique, des éthers d'acide gras polyoxyéthoxylés (Brij®), alkylphénolpoly(oxyéthylène (par exemple Triton X-100®), des phospholipides comme, par exemple, la lécithine ou des dérivés de la lécithine, la cholestérine et des dérivés de la cholestérine, des cholates ou leurs mélanges.

25. Procédé selon la revendication 24, **caractérisé en ce que** l'on utilise, en tant que substances tensioactives :
des phospholipides, l'alcool polyvinylique, des éthers d'acide gras polyoxyéthoxylés (Brij®), des poloxamères (copolymères séquencés polyoxyéthylène-polyoxypropylène), des poloxamines (polymères séquencés polyoxyéthylène-polyoxypropylène de l'éthylènediamine), l'ester éthoxylé d'acide gras de la sorbitane (polysorbates, Tween®), le cholate de sodium et des esters d'acide gras du saccharose ou leurs mélanges.

26. Procédé selon l'une quelconque des revendications 1 à 25, **caractérisé en ce que** l'élimination du solvant ou du mélange de solvants ainsi que, le cas échéant, l'élimination de l'agent actif non capsulé et/ou de l'un ou des agents tensioactifs, se fait dans une filtration à courant transversal ou à l'aide d'un évaporateur à couches minces.

27. Procédé selon l'une quelconque des revendications 1 à 26, **caractérisé en ce qu'**une transition de l'échelle du laboratoire à l'échelle de production est effectuée par une commutation en parallèle de plusieurs micromélangeurs et/ou par l'utilisation de réseaux de mélangeurs (montée en en nombre).

## Claims

1. Continuous process for the production of micro- or nanoparticles from biodegradable synthetic and/or from natural substances, **characterized in that** the production is carried out by means of a micromixer which contains a mixing chamber having microchannels engaging with one another, in which liquid lamellae of the liquids to be mixed are periodically brought to overlap.

2. Process according to Claim 1, **characterized in that** the liquids to be mixed is [sic] a particle-forming liquid phase and a dispersing agent.

3. Process according to Claim 2, **characterized in that** the particle-forming liquid phase is an aqueous or organic solution of a particle-forming substance or a molten particle-forming substance, to which are optionally added surfactants, viscosity enhancers or other stabilizers.

4. Process according to Claim 2, **characterized in that** the dispersion medium is water, an aqueous solution, a hydrophilic liquid, an organic solution or an oily liquid, to which are optionally added a substance inducing coacervation and/or decreasing solubility, surfactants and/or viscosity enhancers.

5. Process according to Claim 2, **characterized in that** the dispersion medium is a single- or multiphase mixture of at least two dispersion media.

6. Process according to one of claims 1 to 5, **characterized in that** the microchannels have a microchannel width of 1 to 1000 µm.

7. Process according to one of Claims 1 to 6, **characterized in that** the walls of the microchannels are arranged in a wavy, parallel or zigzag manner.

8. Process according to one of Claims 1 to 7, **characterized in that** the mixing chamber is an LIEM mixing chamber.

9. Process according to one of Claims 1 to 8, **characterized in that** the particle-forming liquid phase and the dispersion medium are supplied to the micromixer in counter-flow, co-flow or coaxially.

10. Process according to one of Claims 1 to 9, **characterized in that** the particle-forming liquid phase and the dispersion medium are supplied to the micromixer without pressure or at liquid pressures of up to about 30 bar.

11. Process according to one of Claims 1 to 10, **characterized in that** the mixed product is obtained perpendicularly or parallel to the flow direction.

12. Process according to one of Claims 1 to 11, **characterized in that** the process is used for the encapsulation of active compounds.

13. Process according to one of Claims 1 to 12, **characterized in that** the process is used for the production of active compound particles.

14. Process according to one of Claims 1 to 13, **characterized in that** the active compound to be encapsulated is dissolved, suspended or emulsified in the particle-forming liquid phase, directly or dissolved in a suitable medium.

15. Process according to one of Claims 1 to 14, **characterized in that** the emulsification of an active compound or of an active compound solution into the liquid particle-forming phase is carried out by means of a preconnected mixer, preferably micromixer.

16. Process according to one of Claims 1 to 15, **characterized in that** the active compound(s) or the active compound solution is supplied to the micromixer by means of a further inlet bore and the mixing takes place in the micromixer.

17. Process according to one of Claims 1 to 16, **characterized in that** the solvent used is ethanol, isopropanol, methanol, alkyl acetates such as methyl, ethyl, propyl, isopropyl or butyl acetate, alkyl formates such as methyl, ethyl, propyl, isopropyl or butyl formate, triacetin, triethyl citrate and/or C₁-C₄-alkyl lactates, e.g. methyl or ethyl lactate, ketones, e.g. acetone, ethyl methyl ketone.

18. Process according to one of Claims 1 to 17, **characterized in that** the solvents used are methyl acetate, ethyl acetate, propyl acetate, isopropyl acetate and propyl formate.

19. Process according to one of Claims 1 to 18, **characterized in that** the biodegradable synthetic prefabricated polymers employed are polyesters of hydroxycarboxylic acids:
polyglycolides (PGA) and copolymers of glycolides such as glycolide/lactide copolymers (PGA/PLLA [sic] = PLGA) or glycolide/trimethylene carbonate copolymers (PGA/TMC); L-polylactides (PLA) and stereocopolymers of polylactides such as poly-L-lactide (PLLA), poly-DL-lactide copolymers and L-lactide/DL-lactide copolymers; copolymers of PLA such as lactide/tetramethylglycolide copolymers, lactide/δ-valerolactone copolymer and lactide/ε-caprolactone copolymer; poly-β-hydroxybutyrate (PHBA), PHBA/β-hydroxyvalerate copolymers (PHBA/HVA), poly-β-hydroxypropionate (PHPA), poly-p-dioxanone (PDS), poly-δ-valerolactone, hydrophobized polysaccharides, hydrophobized hyaluronic acid, hydrophobized dextrans or hydrophobized amylopectin and poly-ε-caprolactone.

20. Process according to one of Claims 1 to 19, **characterized in that** the biodegradable synthetic polymers employed are block polymers of polyesters of hydroxycarboxylic acids and linear or star polyethylene glycol (PEG):
AB block copolymers of PLA or PLGA and PEG, ABA triblock copolymers of PLA-PEG-PLA or -PLGA, S(3)-PEG-PLA or -PLGA block copolymers and S(4)-PEG-PLA or -PLGA block copolymers.

21. Process according to one of Claims 1 to 20, **characterized in that** the biodegradable synthetic polymers employed are glycolide/lactide copolymers such as Resomer® RG-505, Resomer® RG-756 or Resomer® RG-858 having a lactide/glycolide ratio of 50:50, 75:25, 85:15 or mixtures lying in between and an average molecular weight of between 10,000 and 250,000.

22. Process according to one of claims 1 to 21, **characterized in that** the natural substances used are fats (lipids and lipoids), natural and synthetic mono-, di- and triglycerides, natural and synthetic waxes, hydrocarbons, fatty alcohols and their esters and ethers, lipid peptides, proteins and sugars, and their derivatives and/or their mixtures.

23. Process according to Claim 22, **characterized in that** the natural substances used are:
glycerol trilaurate, myristate, palmitate, stearate, behenate, glycerol trioleate, glycerol monopalmitostearate, cetyl palmitate, coconut oil, stearyl alcohol esters, glycol esters, butanediol esters and glycerol esters of the following fatty acids:
formic, acetic, propionic, butyric, valeric, caproic, oenanthic, caprylic, pelargonic, capric, undecanoic, lauric, tridecanoic, myristic, pentadecanoic, palmitic, margaric, stearic, nonadecanoic, arachic, behenic, lignoceric, cerotic, melissic, isobutyric, isovaleric, tuberculostearic, acrylic, crotonic, palmitoleic, oleic, erucic, sorbic, linoleic, linolenic, elaeostearic, arachidonic, clupanodonic and/or docosahexaenoic acid,
hard paraffin, oleyl alcohol, stearyl alcohol, cetyl alcohol, bleached wax, gelatine, human serum albumin, bovine serum albumin, sodium alginate, chitosan, cellulose, methylcellulose, ethylcellulose, hydroxypropylcellulose, hydroxyethylcellulose, sodium carboxymethylcellulose, pectin, xanthan and starch or their mixtures.

24. Process according to one of claims 1 to 23, **characterized in that** the surface-active substances employed are:
poloxamers (polyoxyethylene-polyoxypropylene block copolymers), poloxamines (polyoxyethylene-polyoxypropylene block polymers of ethylenediamine), polyethylene glycol alkyl ethers, sorbitan fatty acid esters (Span®), ethoxylated sorbitan fatty acid esters (polysorbates, Tween®), sucrose fatty acid esters, gelatine, polyvinylpyrrolidone, fatty alcohol polyglycoside, CHAPS, CHAPSO, decyl β-D-glycopyranoside, decyl β-D-maltopyranoside, dodecyl β-D-maltopyranoside, sodium oleate, polyethylene glycol, polyvinyl alcohol, polyethoxylated fatty acid ethers (Brij®), alkylphenolpoly(oxyethylenes [sic] (e.g. Triton X-100®), phospholipids such as, for example, lecithins or lecithin derivatives, cholesterol and cholesterol derivatives, cholates or their mixtures.

25. Process according to Claim 24, **characterized in that** the surface-active substances employed are:
phospholipids, polyvinyl alcohol, polyethoxylated fatty acids (Brij®), poloxamers (polyoxyethylene-polyoxypropylene block copolymers), poloxamines (polyoxyethylene-polyoxypropylene block polymers of ethylenediamine), ethoxylated sorbitan fatty acid esters (polysorbates, Tween®), sodium cholate and sucrose fatty acid esters or their mixtures.

26. Process according to one of claims 1 to 25, **characterized in that** the removal of the solvent or solvent mixture, and optionally the removal of unencapsulated active compound and/or of the surfactant(s) takes place in a cross-flow filtration or using a thin layer evaporator.

27. Process according to one of Claims 1 to 26, **characterized in that** a scaling-up from the laboratory to a production scale is carried out by the parallel connection of a number of micromixers and/or by the use of mixer arrays (numbering-up).
